# EUROPÄISCHE PATENTSCHRIFT

(11) **EP 0 735 016 B1**
(45) Veröffentlichungstag und Bekanntmachung des Hinweises auf die Patenterteilung: **30.06.1999**
(21) Anmeldenummer: 96104306.4
(22) Anmeldetag: 19.03.1996
(51) Int. Cl.: C07C 45/63

(54) **Verfahren zur Herstellung von Alpha-Chloralkylarylketonen**
Process for the preparation of alpha-chloroalkylarylketones
Procédé pour la préparation d'alpha-chloroalkylarylcétones

(30) Priorität: 31.03.1995 DE 19511861
(43) Veröffentlichungstag der Anmeldung: 02.10.1996
(73) Patentinhaber: BASF Aktiengesellschaft, 67063 Ludwigshafen (DE)
(72) Erfinder: Siegel, Wolfgang, Dr., 67117 Limburgerhof (DE); Dobler, Walter, Dr., 69126 Heidelberg (DE); John, Michael, Dr., 67245 Lambsheim (DE)

(56) Entgegenhaltungen:
- US-A- 4 310 702
- DATABASE WPI Section Ch, Week 8626 Derwent Publications Ltd., London, GB; Class E14, AN 86-165167 XP002007948 & JP-A-61 097 239 (SEITETSU CHEM IND KK) , 15.Mai 1986
- DATABASE WPI Section Ch, Week 9348 Derwent Publications Ltd., London, GB; Class B05, AN 93-383022 XP002007949 & JP-A-05 286 902 (SUMITOMO SEIYAKU KK) , 2.November 1993
- MAGN. RESON. CHEM. (MRCHEG,07491581);87; VOL.25 (2); PP.179-80, UNIV. SAO PAULO;INST. QUIM.; SAO PAULO; 01498; BRAZIL (BR), XP000575702 OLIVATO P R ET AL: "Carbon-13 NMR spectra of some 4-substituted phenacyl chlorides and iodides"

## Beschreibung

Die vorliegende Erfindung betrifft ein neues Verfahren zur Herstellung von α-Chloralkylarylketonen.

α-Chloralkylarylketone sind wertvolle zwischenprodukte für die Synthese von Pharma- und Pflanzenschutzwirkstoffen. Sie lassen sich bisher unter anderem dadurch herstellen, daß Alkylarylketone mit einem Chlorierungsreagens umgesetzt werden.

Von Lands et al. (J. Med. Chemistry 35, 3081 - 3084 (1992)) wird folgende Reaktion beschrieben: 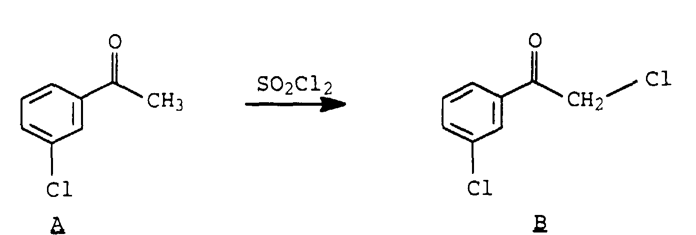 die das Produkt B in 41 % Ausbeute liefert. Eigene Versuche mit SO₂Cl₂ als Chlorierungsreagens zeigen, daß je nach Reaktionsführung entweder unbefriedigende Ausbeuten bzw. zu geringe Selektivitäten bei der Chlorierung erreicht werden.

Es bestand daher die Aufgabe, ein Verfahren zur Herstellung von α-Chloralkylarylketonen durch Chlorierung von Alkylarylketonen mit SO₂Cl₂ bereitzustellen, bei dem die o.g. Nachteile nicht auftreten.

Gefunden wurde ein Verfahren zur Herstellung von α-Chloralkylαrylketonen der allgemeinen Formel I 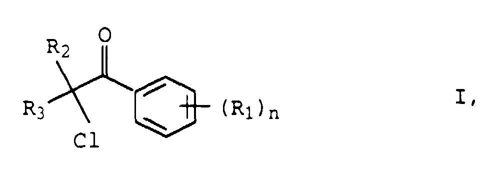 wobei
- n: 1 bis 5,
- R₁: unabhängig voneinander H, Alkyl, Alkoxy, Aryl, Aryloxy, Acyloxy, Acylamino, Halogen, Nitro,
- R_{2,3}: unabhängig voneinander H, Alkyl, Aryl bedeuten,
durch Chlorierung von Alkylarylketonen der allgemeinen Formel II 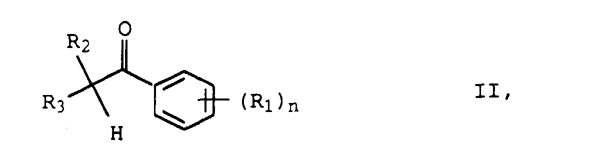 mit Sulfurylchlorid, wobei die Chlorierung in Gegenwart eines aliphatischen Alkohols durchgeführt wird.

Geeignet für das erfindungsgemäße Verfahren sind gesättigte oder ungesättigte aliphatische Alkohole. Bevorzugt werden gesättigte unverzweigte oder verzweigtkettige Alkohole, insbesondere solche mit ein bis zehn C-Atomen verwendet.

Besonders bevorzugte Alkohole sind Methanol, Ethanol, 1-Propanol, 2-Propanol, 1-Butanol, 2-Butanol.

Die Alkohole können sowohl als Einzelsubstanz als auch als Gemische in dem erfindungsgemäßen Verfahren eingesetzt werden.

Gute Ergebnisse lassen sich erzielen, wenn die aliphatischen Alkohole in Mengen von 0,1 Mol bis 10 Mol, bevorzugt von 2 Mol bis 6 Mol, pro Mol Alkylarylketon eingesetzt werden. Niedrigere Molverhältnisse als die angegebenen führen in der Regel zu signifikanten Nebenreaktionen, während höhere Molverhältnisse meist zu unvollständigem Umsatz führen.

Sulfurylchlorid wird in der Regel in einer Menge von 1 Mol bis 2 Mol, bevorzugt 1,0 Mol bis 1,5 Mol, pro Mol Alkylarylketon II eingesetzt.

SO₂Cl₂-Mengen außerhalb dieser Angaben können zwar verwendet werden, sie führen aber zu keiner Verbesserung des erfindungsgemäßen Verfahrens.

Da α-Chloralkylarylketone der Formel I in der Regel Feststoffe sind, wird die Reaktion vorzugsweise in einem Lösungsmittel durchgeführt. Geeignet sind alle Lösungsmittel, die sowohl die eingesetzten Alkylarylketone II als auch die entstehenden α-Chloralkylarylketone I gut lösen und unter den Reaktionsbedingungen keine Reaktion mit Sulfurylchlorid eingehen. Insbesondere geeignet sind aromatische Kohlenwasserstoffe wie Benzol, Toluol, Ethylbenzol, Xylole wie o-Xylol und Tetralin; Halogenaromaten wie Chlorbenzol und Dichlorbenzole, z.B. 1,2-Dichlorbenzol; gesättigte aliphatische Kohlenwasserstoffe der allgemeinen Summenformel CₙH₂ₙ₊₂ mit n = 5 bis 20 wie Hexan, Heptan und Octan sowie chlorierte aliphatische Kohlenwasserstoffe, z.B. Methylenchlorid, Chloroform, Tetrachlormethan, 1,1-Dichlorethan, 1,2-Dichlorethan, 1,1,1-Trichlorethan und 1,1,2-Trichlorethan und Mischungen dieser Lösungsmittel.

Die Reaktion wird vorzugsweise bei Normaldruck durchgeführt, da eine Erhöhung oder Erniedrigung des Drucks keine signifikanten Vorteile hinsichtlich Selektivität, Ausbeute und Verfahrenstechnik mit sich bringt.

Die Reaktion läßt sich in einem Temperaturbereich von -80°C bis +100°C, vorzugsweise im Bereich 0°C bis 50°C ausführen. Reaktionstemperaturen außerhalb dieses Bereichs liefern keine signifikanten Vorteile hinsichtlich Selektivität, Ausbeute und Verfahrenstechnik, führen jedoch wegen des steigenden Energiebedarfs für die Kühlung oder Beheizung zu unnötig hohen Kosten.

Die Reaktion kann in geeigneten Apparaturen sowohl diskontinuierlich als auch kontinuierlich durchgeführt werden.

Die in dem erfindungsgemäßen Verfahren eingesetzten Alkylarylketone der Formel II sind bekannt, kommerziell erhältlich oder lassen sich beispielsweise nach Friedel-Crafts aus aromatischen Kohlenwasserstoffen und Carbonsäurehalogeniden darstellen (Houben Weyl, "Methoden der organischen Chemie", Georg Thieme Verlag, Stuttgart 1973, Band 7/2a, Teil 1, Seiten 23 bis 107 und Seiten 135 - 154).

Die folgenden Beispiele dienen der weiteren Veranschaulichung der Erfindung bzw. zeigen den Stand der Technik auf (Vergleichsbeispiele).

### Beispiel 1

### Allgemeine Arbeitsvorschrift

Es wurden die in Tabelle 1 angegebenen Mengen der in Schema 1 angegebenen Alkylarylketone 1a bis 1d, Alkohole 2a bis 2c sowie die in Tabelle 1 angegebenen Mengen an Sulfurylchlorid, Methylenchlorid und Wasser verwendet. Es wurden die in Tabelle 1 angegebenen Reaktionstemperaturen eingestellt.

Die Alkylarylketone 1a bis 1d wurden in einer Mischung aus Methylenchlorid und einem der aliphatischen Alkohole 2a bis 2c in einer Rührapparatur mit thermostatisiertem Doppelmantel vorgelegt. Die gewünschte Reaktionstemperatur wurde durch Beheizen oder Kühlen der Apparatur mit Hilfe eines Thermostaten eingestellt. Eine Lösung von Sulfurylchlorid in Methylenchlorid wurde unter Beibehaltung der Reaktionstemperatur mit Hilfe einer automatischen Dosiereinrichtung innerhalb 1 Stunde zur gerührten Reaktionsmischung zudosiert. Es wurde eine Stunde bei konstanter Temperatur nachgerührt und anschließend Wasser zur Reaktionsmischung zugegeben. Umsatz und Selektivität der Chlorierungsreaktion wurden durch gaschromatographische Analyse der organischen Phase bestimmt.

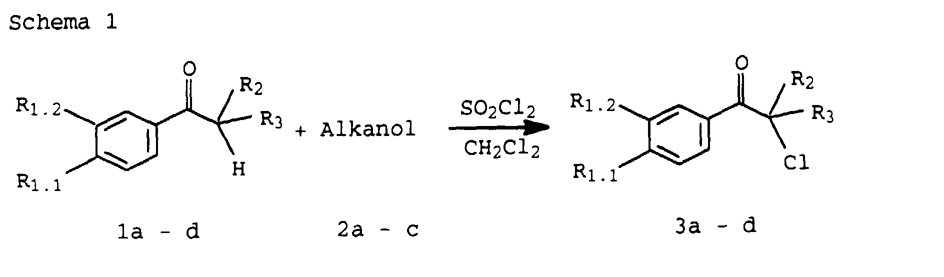

| | R_{1.1} | R_{1.2} | R₂ | R₃ | | | | R_{1.1} | R_{1.2} | R₂ | R₃ |
|---|---|---|---|---|---|---|---|---|---|---|---|
| 1a | H | H | H | H | 2a | Methanol | 3a | H | H | H | H |
| 1b | CH₃ | H | H | H | 2b | Ethanol | 3b | CH₃ | H | H | H |
| 1c | OCH₃ | H | H | H | 2c | 2-Propanol | 3c | OCH₃ | H | H | H |
| 1d | H | H | C₂H₅ | H | | | 3d | H | H | C₂H₅ | H |
| 1e | F | H | H | H | | | 3e | F | H | H | H |
| 1f | OCH₃ | OCH₃ | H | H | | | 3f | OCH₃ | OCH₃ | H | H |

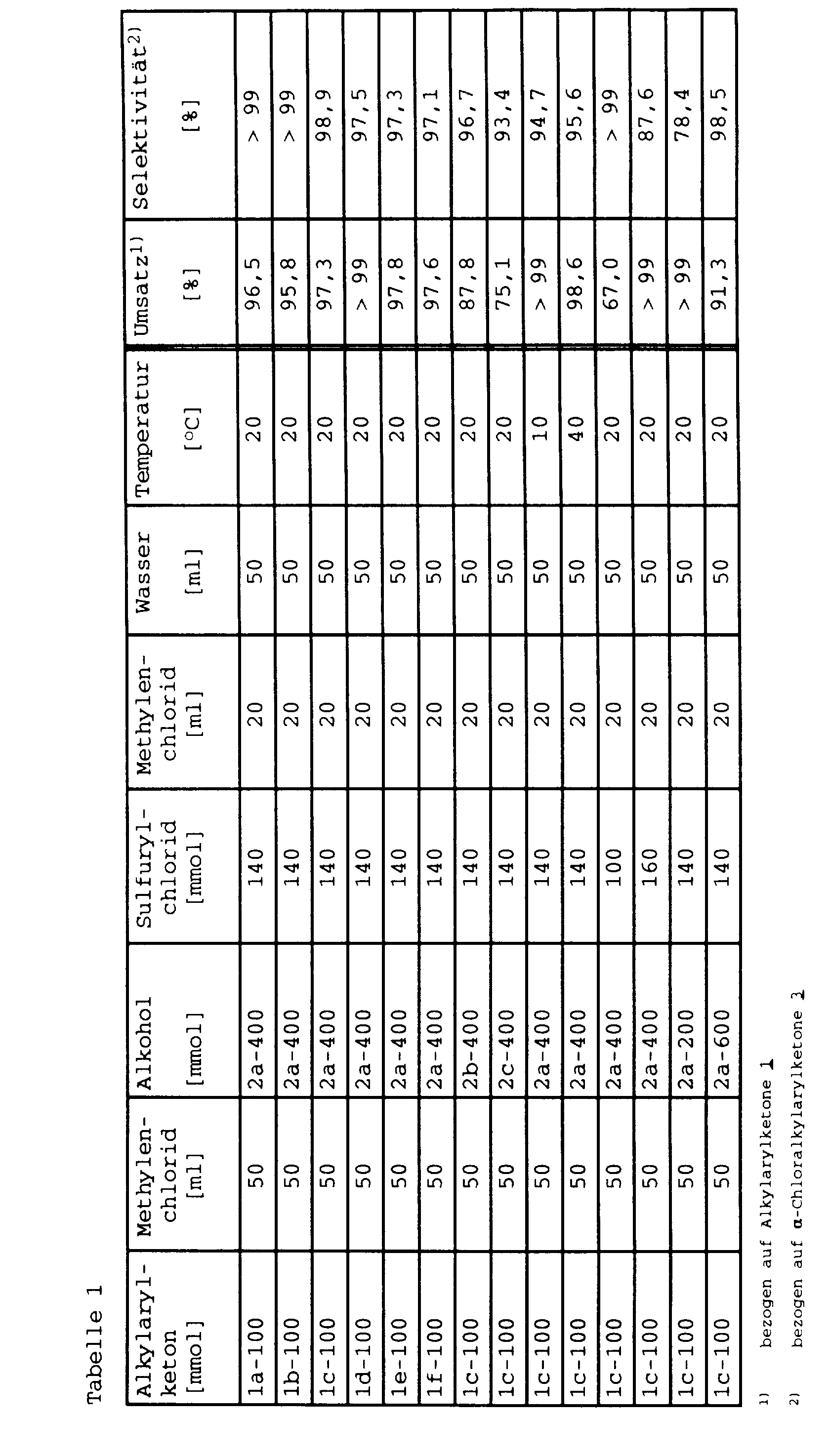

### Beispiel 2 (Vergleichsbeispiel, nicht erfindungsgemäß)

Die Vergleichsexperimente aus Tabelle 2 wurde nach der allgemeinen Arbeitsvorschrift aus Beispiel 1, jedoch ohne Zugabe eines aliphatischen Alkohols durchgeführt. Die Bezifferung bezieht sich auf Schema 1.

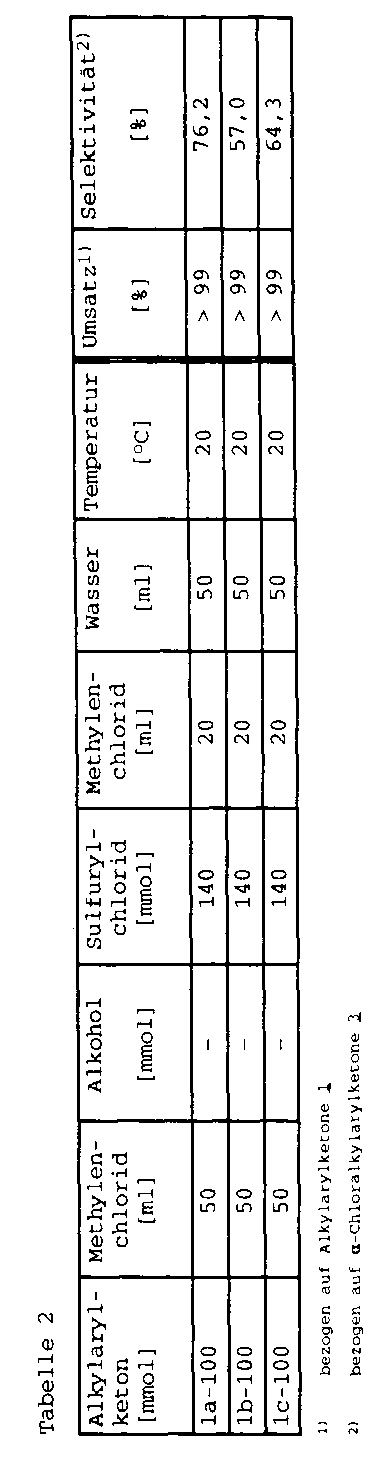

## Patentansprüche

1. Verfahren zur Herstellung von α-Chloralkylarylketonen der allgemeinen Formel I 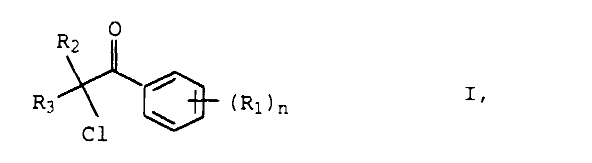 wobei
n 1 bis 5,
R₁ unabhängig voneinander H, Alkyl, Alkoxy, Aryl, Aryloxy, Acyloxy, Acylamino, Halogen, Nitro,
R_{2,3} unabhängig voneinander H, Alkyl, Aryl bedeuten,
durch Chlorierung von Alkylarylketonen der allgemeinen Formel II 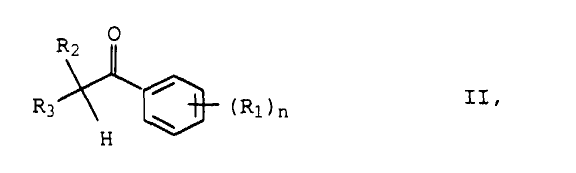 mit Sulfurylchlorid, dadurch gekennzeichnet, daß die Chlorierung in Gegenwart eines aliphatischen Alkohols durchgeführt wird.

2. Verfahren nach Anspruch 1, dadurch gekennzeichnet, daß 0,1 bis 10 Mol Alkohol pro Mol Alkylarylketon II eingesetzt werden.

3. Verfahren nach Anspruch 1 oder 2, dadurch gekennzeichnet, daß ein C₁-C₄-Alkohol eingesetzt wird.

4. Verfahren nach Anspruch 3, dadurch gekennzeichnet, daß als Alkohol Methanol eingesetzt wird.

## Claims

1. A process for preparing α-chloroalkyl aryl ketones of the general formula I 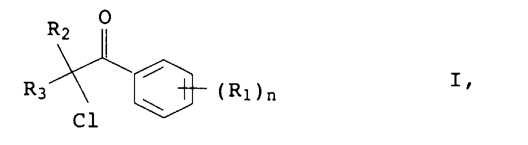 where
n is 1 to 5,
R₁ independently of one another are H, alkyl, alkoxy, aryl, aryloxy, acyloxy, acylamino, halogen or nitro,
R_{2,3} independently of one another are H, alkyl or aryl, by chlorination of alkyl aryl ketones of the general formula II 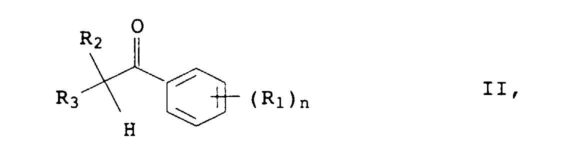 with sulfuryl chloride, which comprises carrying out the chlorination in the presence of an aliphatic alcohol.

2. A process as claimed in claim 1, wherein from 0.1 to 10 mol of alcohol are employed per mole of alkyl aryl ketone II.

3. A process as claimed in claim 1 or 2, wherein a C₁-C₄-alcohol is employed.

4. A process as claimed in claim 3, wherein the alcohol employed is methanol.

## Revendications

1. Procédé pour la préparation d'α-chloroalkylarylcétones de formule générale I 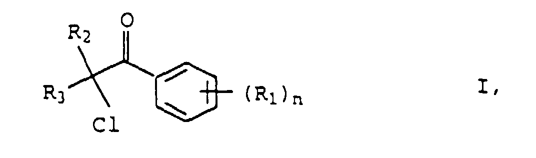 où
n vaut 1 à 5,
R₁ représente, indépendamment l'un de l'autre, H, alkyle, alcoxy, aryle, aryloxy, acyloxy, acylamino, halogéno, nitro,
R_{2,3} représentent, indépendamment l'un de l'autre, H, alkyle, aryle,
par chloration d'alkylarylcétones de formule générale II 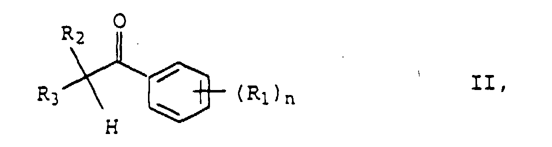 avec du chlorure de sulfuryle, caractérisé par le fait que la chloration est effectuée en présence d'un alcool aliphatique.

2. Procédé selon la revendication 1, caractérisé par le fait qu'on utilise 0,1 à 10 mol d'alcool par mol d'alkylarylcétone II.

3. Procédé selon la revendication 1 ou 2, caractérisé par le fait qu'on utilise un alcool en C₁-C₄.

4. Procédé selon la revendication 3, caractérisé par le fait qu'on utilise du méthanol comme alcool.
